Europäisches Patentamt
European Patent Office
Office européen des brevets

(11) Veröffentlichungsnummer: **0 285 980 A2**

# EUROPÄISCHE PATENTANMELDUNG

(21) Anmeldenummer: **88105048.8**

(22) Anmeldetag: **29.03.88**

(51) Int. Cl.[4] **C07C 43/15 , C07C 43/10 , C11B 9/00**

(30) Priorität: **09.04.87 DE 3712075**

(43) Veröffentlichungstag der Anmeldung: **12.10.88 Patentblatt 88/41**

(84) Benannte Vertragsstaaten: **CH DE FR GB IT LI NL**

(71) Anmelder: **HAARMANN & REIMER GMBH**
**Postfach 138**
**D-3450 Holzminden(DE)**

(72) Erfinder: **Exner, Fritz, Dr.**
**Gartenwinkel 2**
**D-3450 Holzminden(DE)**
Erfinder: **Hagena, Detlef, Dr.**
**Am Feldberg**
**D-3470 Höxter 1(DE)**
Erfinder: **Hopp, Rudolf, Dr.**
**Auf dem Gehrenkamp 28**
**D-3450 Holzminden(DE)**
Erfinder: **Nienhaus, Jürgen, Dr.**
**Eckenbrecherstrasse 20**
**D-3450 Holzminden(DE)**
Erfinder: **Göttsch, Wilhelm**
**Eichenhang 12**
**D-3454 Bevern(DE)**

(74) Vertreter: **Gerhards, Peter, Dr.**
**Bayer AG Konzernverwaltung RP**
**Patentabteilung**
**D-5090 Leverkusen 1 Bayerwerk(DE)**

(54) **Riechstoffe und diese enthaltende Riechstoffkompositionen.**

(57) Die Erfindung betrifft 1,2-Dialkoxyalkene der Formel

$$CH_2=CH-(CH_2)_n-\underset{\displaystyle OR}{\underset{|}{C}}H-CH_2-OR ,$$

in der
R eine Alkylgruppe mit 1 bis 3 Kohlenstoffatomen, und
n eine ganze Zahl von 2 bis 8 bedeutet, und die Verwendung von 1,2-Dialkoxyalkanen und -alkenen der Formel

$$CH_a \overset{-}{-} CH_b - (CH_2)_n - \underset{\displaystyle OR}{\underset{|}{C}}H - CH_2 - OR ,$$

in der R und n die vorstehend angegebene Bedeutung haben und $CH_a \overset{-}{-} CH_b$-die Gruppe $CH_3$-$CH_2$-oder $CH_2=CH$-bedeuten. als Riechstoffe.

EP 0 285 980 A2

## Riechstoffe und diese enthaltende Riechstoffkompositionen

Die Erfindung betrifft neue Riechstoffe und Riechstoffkompositionen, die diese neuen Riechstoffe enthalten.

Es wurde gefunden, daß Verbindungen der allgemeinen Formel

$$CH_a \overset{-}{-} CH_b - (CH_2)_n - \underset{OR}{\underset{|}{CH}} - CH_2 - OR \quad (I),$$

in der R eine Alkylgruppe mit 1 bis 3 Kohlenstoffatomen, n eine Zahl von 2 bis 8 und $CH_a \overset{-}{-} CH_b$-die Gruppe $CH_3$-$CH_2$-oder $CH_2 = CH$-bedeuten, wertvolle Riechstoffe sind und sich zur Herstellung von Riechstoffkompositionen eignen.

Die Erfindung betrifft daher die Verwendung dieser 1,2-Dialkoxyalkane und -alkene der Formel I als Riechstoffe, sowie diese Verbindungen enthaltende Riechstoffkompositionen.

Einige der 1,2-Dialkoxyalkane der Formel I sind als Zwischenprodukte chemischer Reaktionen beschrieben (s. J. Chem. Soc., Perkin Trans. I 1981, 3265; J. Chem. Soc., Perkin Trans. I 1974, 1936; Tetrahedron Lett. 1970, 5275; Tetrahedron Lett. 1983, 4347). Über die sensorischen Eigenschaften dieser Alkane, z.B. ihre Riechstoffeigenschaften, war bislang nichts bekannt.

Die 1,2-Dialkoxyalkene der Formel I sind neue Verbindungen. Die Erfindung betrifft daher auch die Verbindungen der Formel I, in der $CH_a \overset{-}{-} CH_b$ für die $CH_2 = CH$-Gruppe steht. Von diesen neuen Verbindungen zeichnet sich das 1,2-Dimethoxyoct-7-en durch besonders wertvolle Riechstoff-Eigenschaften aus.

Die erfindungsgemäßen Riechstoffe der Formel I können mit Hilfe bekannter Veretherungsverfahren aus den entsprechenden Diolen hergestellt werden (s. z. B. Houben-Weyl, Methoden der organischen Chemie, Sauerstoffverbindugen I, Teil 3, Seiten 24 ff, insbesondere Seiten 33 bis 36). Als besonders vorteilhaft hat sich erwiesen, das Diol mit Natriumhydrid in einem Lösungsmittel wie Toluol oder Xylol in das Natriumalkoholat zu überführen, wobei das Natriumhydrid in einer Menge von 2 bis 2,5 Mol, vorzugsweise 2,2 Mol pro Mol Diol eingesetzt wird. Nach Beendigung der Wasserstoffentwicklung wird dann die Alkylierung mit dem entsprechenen Dialkylsulfat bei Temperaturen zwischen 80 und 150°C, vorzugsweise zwischen 110 und 130°C durchgeführt. Das Dialkylsulfat wird in einer Menge von 2 bis 2,5 Mol, vorzugsweise 2,2 Mol pro Mol Diol eingesetzt.

Der Geruch einiger der erfindungsgemäßen Riechstoffe läßt sich durch die folgenden Geruchsbeschreibungen wiedergeben:

1,2-Dimethoxyhexan:
pilzig, grün, chemisch

1,2-Dimethoxyoctan:
grün, wachsartig, an Flieder, Rose, Reseda und Blattgrün erinnernd

1,2-Diethoxyoctan:
fettig, grün, Olibanum, ranzig

1,2-Dimethoxyoct-7-en:
grün-krautig, Ginster, Galbanum, Gemüse

1,2-Dimethoxydecan:
fettig, aldehydisch

1,2-Dimethoxydodecan:
Orange, fettig, Farnesol

Die erfindungsgemäßen Riechstoffe lassen sich mit anderen, an sich bekannten Riechstoffen, wie sie z. B. im Arctander, Perfume and Flavor Chemicals, Montclair, N.J. (USA) 1969 beschrieben sind, und etherischen Ölen, wie z. B. im Arctander, Perfume and Flavor Materials of natural Origin, Elisabeth, N.J. (USA) 1960 beschrieben sind, zu Riechstoffkompositionen mit ausdrucksstarken Noten kombinieren. Sie eignen sich besonders dazu, die Duftnatürlichkeit künstlicher Blütenöle wie Flieder, Hyazinthe, Jasmin usw. zu erhöhen und in krautigen und würzig-fruchtigen Duftkomplexen den Naturgründuft zu verstärken. Die Dosierung in der Riechstoffkomposition beträgt 0,5 bis 20 Gew.-%, vorzugsweise 1 bis 10 Gew.-% bezogen auf das Gesamtgewicht der Riechstoffkomposition.

Die aus der DE-OS 30 04 661 als Riechstoffe bekannten 1,3-Dioxolane weisen zwar z. T. ähnliche Riechstoffeigenschaften auf, haben aber den Nachteil, daß sie nur in basischem Milieu beständig sind und deshalb auf so wichtigen Anwendungsgebieten wie sauren Haushaltsreinigern, Geschirrspülmitteln, Maschinengeschirrspülmitteln, Kaltwellfixierern, Harrkonditionierern, Haarspülmitteln, Bleich-und Bräunungscremes und Antiperspirants nicht verwendbar sind. Die unter Verwendung der erfindungsgemäßen Verbindungen hergestellten Riechstoffkompositionen eignen sich hervorragend für die Parfümierung von Fertigprodukten des Kosmetik-, Feinparfümerie-, Aerosol-, Waschmittel-und insbesondere des chemisch-technischen Sektors, z. B. für Detergentien, Haarpflegemittel, Schaumbäder, Badesalz, Geschirrspülmittel, Maschinengeschirrspülmittel, Shampoos, Wäscheweichspülmittel, Waschpulver, Seifen, Antiperspirants, Puder, Cremes, Rasierwasser, Aftershave-Lotions, Raumluftverbesserer, WC-Reiniger, Raumsprays, Antiperspirantsprays, Deodorantsprays, Körpersprays, Insektizidsprays und Sonnenschutzmittel.

Die Herstellung der Riechstoffkompositionen und der parfümierten Produkte erfolgt in überlicher Weise, bespielsweise durch Zusammengeben der Komponenten.

Beispiel 1

198 g (6.6 Mol) Natriumhydrid (80%ig) und 500 g Toluol werden vorgelegt und auf Rückflußtemperatur erhitzt. Dann werden innerhalb 3 Stunden 432 g (3 Mol) 7-Octen-1,2-diol, gelöst in 1500 g Toluol, zudosiert und bei Rückflußtemperatur bis zum Ende der Wasserstoffentwicklung nachgerührt. Innerhalb von 4 Stunden werden 830 g (6,6 Mol) Dimethylsulfat zugegeben und weitere 2 Stunden nachgerührt. Nach Zugabe von 50 ml Methanol werden 1500 g Wasser zugegeben, um den Niederschlag zu lösen. Die Phasen werden getrennt und die organische Phase destilliert. Man erhält 420 g 1,2-Dimethoxyoct-7-en mit einem Siedepunkt von 80°C 10 mbar, entsprechend einer Ausbeute von 81 % der Theorie.

Analog wurden hergestellt:

1,2-Dimethoxyhexan: 82 % Ausbeute, Kp: 75°C 30 mbar
1,2-Dimethoxyoctan: 80 % Ausbeute, Kp: 80°C 10 mbar
1,2-Diethoxyoctan : 80 % Ausbeute, Kp: 96°C 10 mbar
1,2-Dimethoxydecan: 84 % Ausbeute, Kp: 90°C 2 mbar
1,2-Dimethoxydodecan: 80 % Ausbeute, Kp: 95°C, 1 mbar

Beispiel 2

Ein Parfümol mit einer Fliedernote wird durch Mischen der folgenden Komponenten hergestellt:

| | |
|---|---|
| Terpineol alpha | 30.0 |
| Hydroxycitronellal | 20.0 |
| Phenylethylalkohol | 15.0 |
| Heliotropin | 10.0 |
| Zimtalkohol | 3.0 |
| Anisaldehyd | 2.0 |
| Linalool | 9.0 |
| Phenylacetaldehyd 50 % in Phenylethylalkohol | 1.0 |
| 1,2-Dimethoxyoctan | 1.0 |
| Dipropylenglycol | 9.0 |
| | 100.0 Gewichtsteile |

Beispiel 3

Ein Parfümol mit einer Orangennote wird durch Mischen der folgenden Komponenten hergestellt:

| | |
|---|---|
| Citral | 4.0 |
| Decylaldehyd | 6.0 |
| Dodecylaldehyd | 0.5 |
| Myristinaldehyd 50% in Diethylphthalat | 0.5 |
| Octylaldehyd | 0.2 |
| Undecylaldehyd | 1.0 |
| Heptanal 10 % in Diethylphthalat | 0.5 |
| Hexanal 10 % in Diethylphthalat | 0.5 |
| Linalool | 5.0 |
| Citronellal | 0.4 |
| Gingergrasöl | 0.2 |
| Nonanal | 0.2 |
| Nonanol | 1.0 |
| Trimethylundecylenaldehyd | 1.0 |
| Orangenöl (brasilianisch) | 50.0 |
| Terpineol alpha | 2.0 |
| Myrcen | 0.5 |
| Carvon | 1.0 |
| Nerol | 1.0 |
| Nerylacetat | 1.0 |
| 1.2-Dimethoxyoctan | 10.0 |
| Dipropylenglycol | 13.5 |
| | 100.0 Gewichtsteile |

## Ansprüche

1. Verwendung von 1,2-Dialkoxyalkanen und -alkenen der Formel

$$CH_a \overset{-}{-} CH_b - (CH_2)_n - \underset{OR}{CH} - CH_2 - OR$$

in der
R eine Alkylgruppe mit 1 bis 3 Kohlenstoffatomen,
n eine ganze Zahl von 2 bis 8 und
$CH_a \overset{-}{-} CH_b$-die Gruppe $CH_3$-$CH_2$-oder $CH_2 = CH$-bedeuten, als Riechstoffe.

2. Riechstoffkompositionen, gekennzeichnet durch einen Gehalt an 1.2-Dialkoxyalkanen oder -alkenen nach Anspruch 1.

3. 1.2-Dialkoxyalkene der Formel

$$CH_2=CH-(CH_2)_n-\underset{OR}{\underset{|}{CH}}-CH_2-OR$$

in der
R eine Alkylgruppe mit 1 bis 3 Kohlenstoffatomen, und
n eine ganze Zahl von 2 bis 8 bedeutet.

4. 1.2-Dimethoxyoct-7-en.